# FASCICULE DE BREVET EUROPEEN

(11) **EP 4 194 015 B1**
(45) Date de publication et mention de la délivrance du brevet: **26.06.2024**
(21) Numéro de dépôt: 22212332.5
(22) Date de dépôt: 08.12.2022
(51) Int. Cl.: A61L 15/18, A61L 15/22, A61L 15/42, A61L 15/44, A61L 15/46

(54) **COMPOSITION DE TISSU POUR SON UTILISATION POUR UNE ACTIVITÉ BACTÉRICIDE, VIRUCIDE, FONGICIDE AINSI QUE POUR LA RÉGÉNÉRATION TISSULAIRE ORGANIQUE AU NIVEAU D'UNE LÉSION**
GEWEBEZUSAMMENSETZUNG FÜR IHRE VERWENDUNG FÜR BAKTERIZIDE, VIRUZIDE, FUNGIZIDE AKTIVITÄTEN SOWIE FÜR ORGANISCHE GEWEBEREGENERATION AM ORT EINER LÄSION
TISSUE COMPOSITION FOR ITS USE FOR BACTERICIDAL, VIRUCIDAL, FUNGICIDAL ACTIVITY AS WELL AS FOR ORGANIC TISSUE REGENERATION AT THE SITE OF A LESION

(30) Priorité: 09.12.2021 FR 2113196
(43) Date de publication de la demande: 14.06.2023
(73) Titulaire: Christine Nayrac Innovation, 31800 Landorthe (FR); Centre National de la Recherche Scientifique, 75016 Paris (FR); Université de Montpellier, 34090 Montpellier (FR)
(72) Inventeur: de CHABALIER NAYRAC, Christine, 31800 LANDORTHE (FR); KISSA, Karima, 34090 MONTPELLIER (FR)
(74) Mandataire: Ipside

(56) Documents cités:
- ALVEN SIBUSISO ET AL: "Electrospun Nanofibers/Nanofibrous Scaffolds Loaded with Silver Nanoparticles as Effective Antibacterial Wound Dressing Materials", PHARMACEUTICS, vol. 13, no. 7, 26 juin 2021 (2021-06-26), page 964, XP055945829, DOI: 10.3390/pharmaceutics13070964
- ABDULLAH AL-ENIZI ET AL: "Polymer-Based Electrospun Nanofibers for Biomedical Applications", NANOMATERIALS, VOL. 8, N.4, vol. 8, no. 4, 20 avril 2018 (2018-04-20), pages 1-22, XP055686219, DOI: 10.3390/nano8040259

## Description

### Domaine technique de l'invention

L'invention relève des tissus aux propriétés bactéricides, virucides et fongicides mais aussi à la propriété régénératrice de tissus organiques notamment au niveau d'une lésion. Elle s'inscrit dans le domaine des compositions de vêtements ou de matériel comprenant un tissu, et concerne plus particulièrement une composition de tissu et son utilisation pour une activité bactéricide, virucide, fongicide, un vêtement comportant un tel tissu, l'utilisation de la composition de tissu pour la régénération de tissus organiques lésés.

### Technique antérieure

La crise sanitaire du COVID avec la production de masques en tissu catégorie 1 a permis une véritable réflexion au niveau de la recherche et du développement sur les tissus bactéricides, virucides et fongicides.

Un tissu comprend habituellement au moins un fil de chaîne et un fil de trame, ce qui permet de dire de l'agencement de ces fils qu'il est tissé et forme donc un tissu, contrairement à des agencements de fils élaborés de manière différente comme par tricotage formant ainsi une maille. Le nom de fil de chaîne est souvent attribué au fil vertical, et le nom de fil de trame au fil horizontal.

Aujourd'hui, on peut trouver dans le secteur de la santé des tissus lavables recouverts d'un film biocide et/ou virucide, notamment dans la fabrication des masques de protection du nez et de la bouche. Cette solution, bien qu'efficace, ne l'est pas que pour une durée limitée car le film biocide se délite après quelques lavages. De plus, les solvants utilisés pour la fabrication du film appliqué sur le tissu ont souvent l'inconvénient d'être allergisants.

Il existe également des tissus dits antiprojections qui permettent d'éviter que l'inoculum viral ou des projections bactériennes se fixent au tissu. Cette propriété est conférée au tissu grâce à une finition déperlante aux projections et aérosols. Cette finition est également fragile dans le temps et se délite après environ 80 lavages ou moins dans certains cas.

Il est également connu de l'art antérieur des tissus comprenant des ions argent (Ag⁺) dans le polymère du fil. Les ions argent sont connus pour leur effet bactéricide. Ces tissus ont un effet bactéricide pérenne. De tels tissus sont notamment utilisés en surface de certains pansements.

Il existe encore aujourd'hui des besoins d'amélioration des tissus bactéricides, virucides et fongicides existants.

Les documents suivants décrivent des compositions de tissu pour pansement, comprenant du fil en polymère dans lequel des ions argent sont intégrés :
1. Alven Sibusiso et al., "Electrospun Nanofibers/Nanofibrous Scaffolds Loaded with Silver Nanoparticles as Effective Antibacterial Wound Dressing Materials", PHARMACEUTICS, vol. 13, no. 7, 26 juin 2021, page 964.
2. Abdullah Al-Enizi et al., "Polymer-Based Electrospun Nanofibers for Biomédical Applications", NANOMATERIALS, vol. 8, no. 4, 20 avril 2018, pages 1-22

### Présentation de l'invention

La présente invention a pour objectif de palier les inconvénients précités en proposant une composition de tissu caractérisée en ce qu'elle comprend :
- au moins 50%, de préférence au moins 55%, de préférence au moins 60% de fil en polymère comprenant au moins 1% de nanoparticules de céramique par rapport à la masse totale du fil, lesdites nanoparticules céramiques étant choisies parmi des nanoparticules de dioxyde de manganèse, d'alumine, d'oxyde de cobalt III, d'oxyde de fer III et de dioxyde de titane ou un mélange d'au moins deux de celles-ci ;
- du fil en polymère dans lequel au moins 100 ppm d'ions argent sont intégrées.

Les inventeurs ont découvert qu'un tissu ayant cette composition associant du fil en polymère dans la masse duquel au moins 100 ppm d'ions argent sont intégrées avec du fil polymère dans la masse duquel est intégré au moins 1% de nanoparticules de céramique, a des propriétés bactéricides, virucides et fongicides accrues par rapport à celles des tissus de l'art antérieur et ses propriétés sont conservées par le tissu pendant une durée supérieure à la durée de conservation de ces propriétés chez les compositions de tissu de l'art antérieur. Les nanoparticules de céramiques sont intégrées dans le polymère du fil ce qui permet de garder lesdites propriétés très longtemps, notamment au-delà de 200 lavages, le tissu étant lavable. La composition de tissu objet de la présente invention permet notamment de se passer de finition déperlante pour le tissu, une telle finition comprenant souvent des solvants aux effets allergènes.

Selon un autre aspect, la présente invention vise un vêtement ou accessoire comprenant la composition de tissu objet de la présente invention, de préférence constitué majoritairement de la composition de tissu objet de la présente invention. On entend par majoritairement que le vêtement ou accessoire comprend la composition de tissu objet de la présente invention à hauteur d'une quantité supérieure à 50% par rapport à sa composition totale. De tels vêtements comprenant la composition de tissu objet de la présente invention sont particulièrement avantageux pour la prévention des infection nosocomiales. On entend par accessoires, notamment les gants, les bonnets, les chaussures, les sur-chaussures, les masques de protection, housse de siège (notamment sièges de voitures), housse de table, draps, alèses, etc.

Selon un autre aspect, la présente invention vise un équipement de protection individuelle comprenant la composition de tissu objet de la présente invention. Les équipements de protection individuelle (EPI) sont très utilisés dans les établissements de santé et comprennent notamment les blouses, les blouses de bloc opératoire, les masques de protection, les gants, les bonnets, etc... La quasi-totalité des EPI des établissements de santé sont aujourd'hui en polyester/coton qui ne vont pas au-delà de 80 lavages et sans effet bactéricide, virucide et fongicide. Les EPI comprenant la composition de tissu objet de la présente invention ont pour avantage d'avoir un effet bactéricide, virucide et fongicide, et de plus, ils résistent et conservent ces dernières propriétés après plus de 200 lavages. Ils présentent donc un net avantage économique et réduisent les risques d'infections nosocomiales.

Selon un autre aspect, la présente invention vise un pansement ou un bandage comprenant la composition de tissu objet de la présente invention.

La présente invention vise également la composition de tissu, le vêtement, l'accessoire, l'équipement de protection individuelle, le pansement ou le bandage objet de la présente invention, pour son utilisation en tant que bactéricide et/ou virucide et/ou fongicide.

La présente invention vise également la composition de tissu, le vêtement, l'accessoire, l'équipement de protection individuelle, le pansement ou le bandage objet de la présente invention, pour son utilisation dans la prévention d'infection nosocomiale chez le mammifère.

De plus, les inventeurs ont découvert que la composition objet de la présente invention a des propriétés permettant avantageusement d'améliorer la régénération de tissus organiques lésés. Cette composition de tissu permet notamment et avantageusement de favoriser le recrutement des macrophages au niveau d'une lésion de tissus organiques, de faciliter la régénération des chondrocytes et la régénération nerveuse (nerfs sensoriels et moteurs) notamment au niveau du système nerveux sympathique et parasympathique.

A cet effet, la présente invention vise également la composition de tissu, le pansement ou le bandage objet de la présente invention pour son utilisation dans le traitement d'une lésion d'un tissu organique, notamment chez le mammifère. Les inventeurs ont remarqué que de façon surprenante et avantageuse, la composition objet de la présente invention entraine une augmentation significative d'au moins 25% de la régénération de tissus organiques ayant subi une lésion par rapport à la régénération de tissu organiques lésés non traité avec la composition objet de la présente invention.

Les inventeurs ont également relevé que de manière avantageuse, la composition objet de la présente invention améliore avantageusement le recrutement de macrophages au niveau de tissus organiques lésés par rapport au recrutement de de macrophages au niveau tels tissus organiques en absence de la composition objet de la présente invention.

A cet effet, dans des modes de réalisation particulier, la présente invention vise également la composition de tissu, le pansement ou le bandage objet de la présente invention pour son utilisation dans recrutement de macrophages au niveau d'une lésion de tissus organiques chez le mammifère.

Les inventeurs ont aussi constaté que de manière avantageuse, la composition objet de la présente invention améliore avantageusement la régénération des chondrocytes par rapport à leur régénération en absence de la composition objet de la présente invention. Il en est de même pour la régénération nerveuse des nerfs sensoriels et moteurs (système nerveux sympathique et parasympathique) par rapport à leur régénération en absence de la composition objet de la présente invention.

A cet effet, dans des modes de réalisation particulier, la présente invention vise également la composition de tissu, le pansement ou le bandage objet de la présente invention pour son utilisation dans la régénération des chondrocytes.

Dans des modes de réalisation particulier, la présente invention vise également la composition de tissu, le pansement ou le bandage objet de la présente invention pour son utilisation dans la régénération nerveuse.

Dans des modes particuliers de réalisation, l'invention répond en outre aux caractéristiques suivantes, mises en oeuvre séparément ou en chacune de leurs combinaisons techniquement opérantes.

Dans des modes de réalisation particuliers, le fil en polymère comprenant les nanoparticules céramiques comprend entre 1% et 3%, de préférence au moins 3%, préférentiellement 3%, de nanoparticules céramiques. En combinaison avec du fil en polymère dans lequel au moins 100 ppm d'ions argent sont intégrées, une quantité de 1% de nanoparticules de céramique dans la masse totale du fil polymère comprenant lesdites particules confère au tissu comprenant ces deux types de fils la propriété avantageuse de contenir et limiter la croissance de contaminants tels que des virus, des bactéries et des champignons parasites avec lesquels il est en contact. De plus, ce tissu avec de telles quantités d'ions argent et de nanoparticules de céramique, permet un recrutement de macrophages important au niveau d'une lésion de tissus organiques avec lesquels il est en contact, et ainsi a pour avantage de favoriser et accélérer la régénération des tissus organiques lésés. Lorsque le fil en polymère comprenant les nanoparticules céramiques comprend entre 1% et 3% de nanoparticules céramiques, associées au fil en polymère dans lequel au moins 100 ppm d'ions argent sont intégrées, ces quantités ont pour avantage de permettre de contenir et limiter encore plus la croissance de contaminants avec lesquels le tissu est en contact. Ces quantités permettent également un recrutement de macrophages encore plus important au niveau d'une lésion de tissus organique et une amélioration plus importante de la régénération des tissus organiques lésés. Au-delà de 3% de nanoparticules céramiques dans le fil polymère comprenant lesdites particules, ledit fil étant associé au fil polymère comprenant au moins 100 ppm d'ions argent, le gain pour le tissu sur la limitation de croissance des contaminants n'évolue que très peu et le recrutement de macrophages n'est pas aussi important qu'avec des quantités de nanoparticules entre 1% et 3%, ce qui n'améliore pas la régénération des tissus lésés par rapport à la régénération tissulaire obtenue avec de telles quantités. En-dessous de 1% de nanoparticules céramiques dans le fil polymère comprenant lesdites particules, ledit fil étant associé au fil polymère comprenant au moins 100 ppm d'ions argent, la limitation de croissance des contaminants et le recrutement de macrophages sont beaucoup moins importants (régénération tissulaire au niveau d'une lésion moins efficace) qu'avec des quantités de nanoparticules entre 1 % et 3%.

Selon l'invention, les nanoparticules céramiques sont choisies parmi des nanoparticules de dioxyde de manganèse, l'alumine, l'oxyde de cobalt III, l'oxyde de fer III et le dioxyde de titane ou un mélange d'au moins deux de celles-ci.

Dans des modes de réalisation particuliers de la présente invention, le fil en polymère comprenant les ions argent, comprend 100 ppm d'ions argent.

Dans des modes préférés de réalisation de l'invention, la composition de tissu comporte au moins 50 %, de préférence au moins 55%, préférentiellement au moins 60% de fil de polymère comprenant les nanoparticules de céramique et le fil restant étant du fil de polymère comprenant les ions argent. C'est dans de telles proportions que la composition obtient des propriétés bactéricides, virucides, fongicides et régénératrice de tissus organiques les plus efficaces.

Dans des modes particuliers de réalisation de l'invention, la composition de tissu comporte entre 50% et 70%, de préférence entre 55% et 65%, préférentiellement entre 60% et 65% de fil de polymère comprenant les nanoparticules de céramique.

Dans des modes particuliers de réalisation de l'invention, la composition de tissu comporte entre 30% et 50%, de préférence entre 35% et 45%, préférentiellement entre 35% et 40% de fil de polymère comprenant les ions argent.

Dans des modes préférés de réalisation de l'invention, la composition de tissu comporte 63% de fil de polymère comprenant les nanoparticules de céramique et 37% de fil de polymère comprenant des ions argent. La composition selon l'invention avec ces proportions permet d'obtenir un des meilleurs résultats concernant ses propriétés bactéricides, virucides, fongicides et régénératrice de tissus organiques.

Dans des modes particuliers de réalisation de l'invention, le fil de polymère comprenant les nanoparticules de céramique constitue la chaîne du tissu et le fil polymère comprenant les ions argent constitue la trame du tissu, ou inversement, le fil de polymère comprenant les nanoparticules de céramique constitue la trame du tissu et le fil polymère comprenant les ions argent constitue la chaîne du tissu. Cette répartition des différents fils permet d'obtenir les effets avantageux de la composition de tissu de manière homogène sur toute la surface du tissu.

Dans des modes de réalisation particulier, le polymère du fil comprenant des nanoparticules de céramique et/ou du fil comprenant les ions argent sont choisis parmi du polyester et du polyamide. Le polymère de polyester peut provenir de recyclage de plastiques et a donc un intérêt écologique avantageux.

### Brève description des figures

L'invention sera mieux comprise à la lecture de la description suivante, donnée à titre d'exemple nullement limitatif, et faite en se référant aux figures qui représentent :

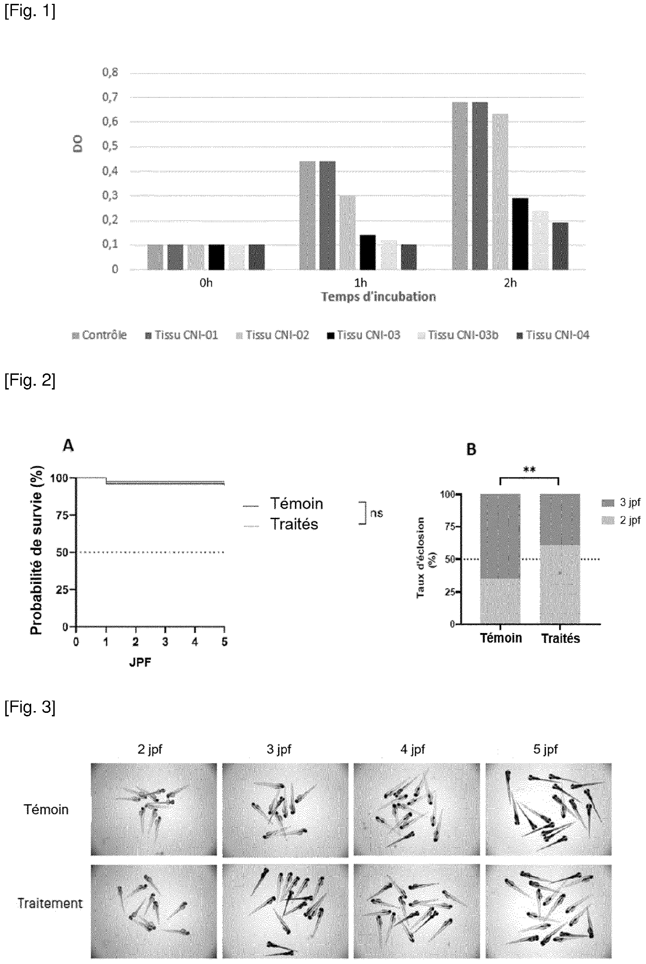

[Fig. 1] la figure 1 est un graphe exposant la croissance de Staphylocoque aureus (*staphylococcus aureus*) en milieu de culture bouillon de soja tryptique (TSB pour « *Tryptic Soy Broth »* en terminologie anglo-saxonne) notamment au contact de compositions de tissu objet de la présente invention (CNI-03b, CNI-04), en fonction du temps, obtenue par analyse de la densité optique du milieu de culture dans le temps ;
[Fig. 2] la figure 2 illustre la survie et l'éclosion d'embryons de poissons zèbre au contact de la composition de tissu objet de la présente invention, avec en A une courbe représentant l'analyse (selon le test statistique Mantel-Cox) de la survie des larves traitées et témoins, et en B un histogramme représentant l'analyse (selon le test statistique Mann-Whitney) de l'éclosion des oeuf traités et témoins ;
[Fig. 3] la figure 3 illustre des images de larves de poissons zèbre témoins et traitées avec la composition de tissu objet de la présente invention, observées au microscope en lumière transmise à 2, 3, 4 et 5 jours post-fécondation, pour déterminer le potentiel toxicité aigüe de ladite composition de tissu ;

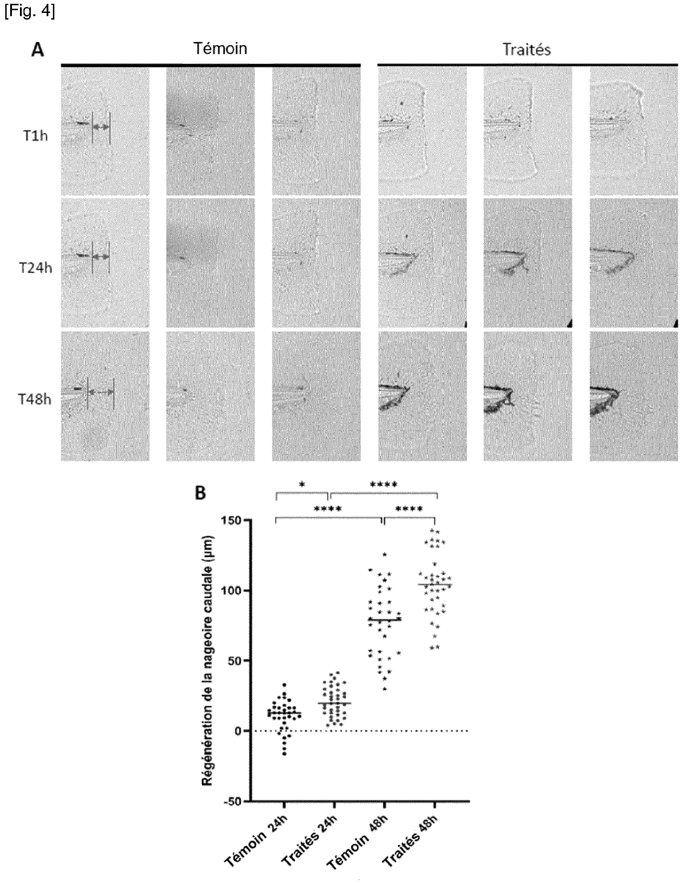

[Fig. 4] la figure 4 illustre en A des images de la régénération dans le temps de la nageoire caudale de larves de poissons zèbre traitées ou non avec la composition de tissu objet de la présente invention, observée à la loupe binoculaire, et en B un graphe résultant d'une analyse statistique (test statistique one-way ANOVA) de la régénération en µm dans le temps de la nageoire caudale de larves de poissons zèbre traitées ou non avec ladite composition de tissu.

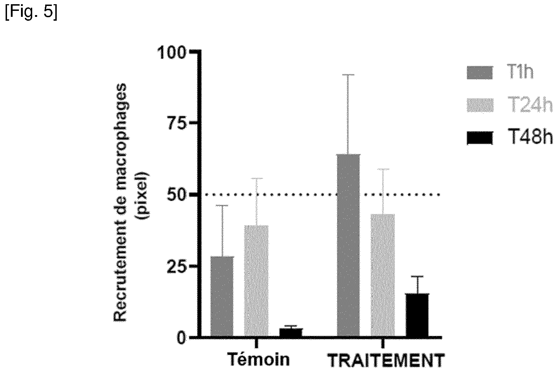

[Fig. 5] la figure 5 illustre un graphe montrant le recrutement de macrophages (pixel) au niveau de la région d'une blessure causée par amputation, à 1 heure (T1h), 24 heures (T24h) et 48 heures (T48h) post-amputation, chez les larves de poissons zèbre traitées ou non avec la composition de tissu objet de la présente invention.

Pour des raisons de clarté, les dessins ne sont pas à l'échelle, sauf mention contraire.

### Description des modes de réalisation

Les expériences suivantes apportent des informations démontrant l'efficacité de compositions de tissu objet de la présente invention lors de leur utilisation en tant que bactéricide, ainsi que lors de leur utilisation dans la régénération de tissus organiques lésés.

Les compositions de tissu objet de la présente invention sont identifiées dans le reste de la description ci-dessous sous la dénomination « tissu CNI-03b » et « tissu CNI-04 ».

Selon un mode de mise en oeuvre, le tissu CNI-04 est fabriqué selon un procédé comprenant les étapes suivantes de :
- polymérisation et création de filaments de polymère de polyester à partir par exemple de pétrole brut après raffinage ou à partir de matière plastique recyclée avec intégration dans la masse du polymère de nanoparticules de céramique de sorte que le polymère emprisonne lesdites nanoparticules de céramique les empêchant ainsi par la suite de migrer hors du polymère ;
- assemblage de plusieurs filaments de polymère de polyester comprenant les nanoparticules de céramique intégrées de sorte à former un fil en polymère (filage) comprenant 3% de nanoparticules de céramique par rapport à la masse totale du fil ;
- tissage du tissu CNI-04 avec au moins 50% de fil en polymère comprenant 3% de nanoparticules de céramique et du fil en polymère de polyester dans la masse duquel 100 ppm d'ions argent Ag⁺ sont intégrées, le fil en polymère comprenant les nanoparticules de céramique constituant la chaîne du tissu et le fil polymère comprenant les ions argent constituant la trame du tissu, ou inversement, le fil de polymère comprenant les nanoparticules de céramique constituant la trame du tissu et le fil polymère comprenant les ions argent constituant la chaîne du tissu.

Le tissu CNI-03b est fabriqué de la même manière à la seule différence que lors du filage, ce dernier est effectué de sorte que le fil en polymère comprend 1% de nanoparticules de céramique par rapport à la masse totale du fil.

### Etude des propriétés bactéricides de la composition de tissu

Les inventeurs ont réalisé une étude comparée de croissance de souche bactérienne *Staphylococcus aureus* (GFP) dans le temps en présence de quatre tissus de compositions différentes :
- un tissu CNI-01 dont la composition comprend seulement du fil de polymère polyester dans lequel 100 ppm d'ions argent sont intégrées, et une finition déperlante en résine téflon.
- un tissu CNI-02 dont la composition est la même que CNI-01 sans la finition déperlante.
- un tissu CNI-03 dont la composition comprend seulement du fil de polymère polyester dans lequel sont intégrées 1% de nanoparticules de céramique de dioxyde de titane, dioxyde de manganèse, alumine et oxyde de fer III.
- un tissu CNI-03B dont la composition comprend en chaîne du fil de polymère polyester dans lequel sont intégrées 1% de nanoparticules de céramique de dioxyde de titane, dioxyde de manganèse, alumine et oxyde de fer III, par rapport à la masse totale du fil, et en trame du fil de polymère polyester dans lequel 100 ppm d'ions argent sont intégrées.
- un tissu CNI-04 dont la composition comprend en chaîne du fil de polymère polyester dans lequel sont intégrées 3% de nanoparticules de céramique de dioxyde de titane, dioxyde de manganèse, alumine et oxyde de fer III, par rapport à la masse totale du fil et en trame du fil de polymère polyester dans lequel 100 ppm d'ions argent sont intégrées.

Chaque tissu est stérilisé par autoclave à sec. Pour faciliter les expériences, le tissu peut avoir été broyé en amont de la stérilisation de sorte à obtenir une poudre de tissu.

Pour chaque tissu, il est réalisé un ensemencement de 2 mL de milieu de culture bouillon de soja tryptique (TSB) en tube à essai avec une souche *staphylococcus aureus* (NSA735-GFP) colonisante et virulente (pied du diabétique surinfecté) avec des concentrations très élevées (de l'ordre de 10⁸/ml) puis une mise en culture en présence du tissu. Un contrôle est également réalisé avec un ensemencement et mise en culture sans tissu dans tube à essai. Une incubation à 37°C avec agitation est réalisée. Le milieu de culture TSB est un milieu nutritif qui permet la croissance d'un grand nombre de microorganismes, en particulier les bactéries. La densité optique (660 nm) du contenu du contenu de chaque tube est mesurée toutes les heures pendant quatre heures. Il est ainsi possible de connaître la croissance de *Staphylococcus aureus* au cours du temps. Les résultats sont présentés en figure 1.

Les résultats montrent que la croissance de la souche de *Staphylococcus aureus* est ralentie au contact du tissu CNI-03 et encore plus ralentie au contact des tissus CNI-03b et CNI-04, ces derniers étant de compositions de tissu objet de la présente invention.

### Etude d'écotoxicité de la composition de tissu

Cette étude a pour objectif d'identifier la toxicité aigüe ou létale de la composition objet de la présente invention sur le poisson-zèbre (*Danio rerio*) au stade embryonnaire. Le protocole suit la ligne directrice de l'Organisation de coopération et de développement économiques (OCDE) 236.

Des oeufs de poisson-zèbre à 6 heures post-fécondation (hpf) sont exposés au tissu CNI-04 durant une période de 120 heures dans une boite de Pétri de 10cm.

Le groupe traité, exposé au tissu CNI-04 est constitué de 78 individus et le groupe témoin non exposé, est composé de 78 individus.

Toutes les 24 heures, quatre observations sont effectuées comme indicateurs de létalité : (a) la coagulation des oeufs fécondés, (b) l'absence de formation de somites, (c) le non-détachement du bourgeon caudal dans le sac vitellin, (d) l'absence de battements du coeur.

Les résultats sont indiqués en figure 2 (A : Analyse de la survie des larves traitées et témoin avec test statistique Mantel-Cox, B : Analyse de l'éclosion des oeufs traités et témoins avec test statistique mann-Whitney) et dans le tableau 1 ci-dessous :

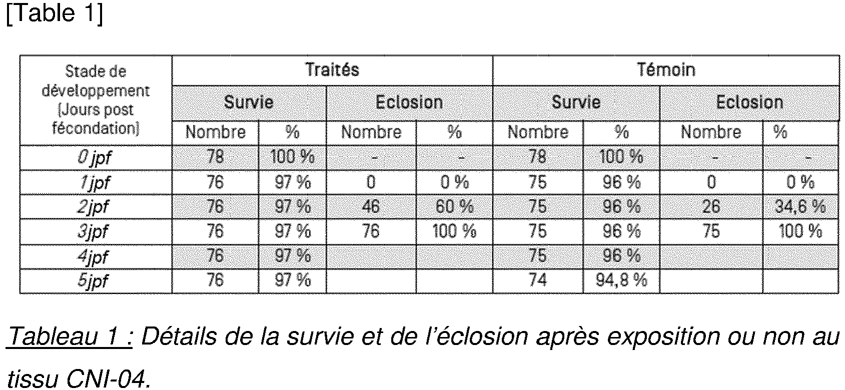

Les résultats apportent les conclusions suivantes :
- mortalité : aucune différence significative sur le taux de survie n'est observée entre les deux conditions (traités et témoins) ;
- taux d'éclosion : le tissu CNI-04 induit un taux d'éclosion plus élevé à 2jpf (60%) par rapport au groupe témoins (34,6%). A 3 dpf, 100% des oeufs ont éclos dans les deux conditions (traités et témoin) ;
- défauts de développement : aucun défaut de développement n'a été observé dans les conditions testées (traités et témoin).

La composition de tissu objet de la présente invention ne présente donc pas d'écotoxicité.

### Etude de toxicité aigüe de la composition de tissu

Cette étude a pour objectif d'évaluer la survie de larves de poisson-zèbre (*Danio rerio*) et la nature des effets toxiques à la suite d'une exposition à la composition de tissu objet de la présente invention.

Les larves à 1 jour post fécondation (jpf) sont exposées au tissu CNI-04 durant une période de 4 jours dans une boite de Pétri de 10cm.

Le groupe traité, exposés au tissu est constitué de 50 individus et le groupe témoin non exposé, est composé de 50 individus.

Toutes les 24 heures, plusieurs observations des larves au microscope en lumière transmise sont réalisées et différents indicateurs d'anomalies sont surveillés (oedèmes, hémorragies, défauts de la circulation sanguin, nécrose...). Les résultats sont illustrés en figure 3 qui représente des images observées au microscope en lumière transmise chez les larves témoins et les larves traitées dans le temps (2, 3, 4 et 5 jours post-fécondation).

Les résultats montrent qu'aucun défaut de développement n'est observé dans aucun groupe (témoins et traités). La composition de tissu objet de la présente invention ne présente donc pas de toxicité aigüe.

### Etude de la régénération après amputation de la nageoire caudale

Un modèle de blessure ouverte après une amputation partielle de la nageoire à l'aide d'un scalpel, affectant notamment le derme et le chondrocyte, reproduit les effets régénératifs observés sur une plaies ouvertes chez l'homme, en condition normale et en condition d'infection bactérienne. Ce modèle est utilisé pour la suite de l'étude.

Cette étude a pour objectif d'identifier l'impact de la composition de tissu objet de la présente invention sur la régénération de tissus organiques chez les larves de *Danio rerio.*

Les larves sont traitées avec du PTU (1-phenyl 2-thiourea) à partir de un jour post-fécondation (1 jpf). Cette substance inhibitrice de la mélanogenèse facilite l'observation de la régénération de la nageoire caudale.

A trois jours post-fécondation (3 jpf), une partie de la nageoire caudale des larves est amputée et les larves sont placées dans des plaques 96 puits comprenant de l'eau, une larve par puit, afin d'observer la régénération. 37 larves ont été traitées avec des fils de tissu CNI-04 dilués dans l'eau des puits. Le groupe témoin composé de 34 larves n'a pas été traité avec des fils de tissu. Les puits sont ensuite incubés à 28°C pendant 24 ou 48 heures.

Les puits sont ensuite rincés plusieurs fois de sorte à éliminer les fils et permettre une visualisation des résultats sous loupe binoculaire.

Les résultats sont illustrés en figure 4 qui montre :
- en A des images de la régénération de la nageoire caudale à 1 heure post-amputation (T1h), 24 heures post-amputation (T24h) et 48 heures post-amputation (T48h) ;
- en B un graphe de la régénération de la nageoire caudale des larves (µm) en fonction du temps (heures) suie à un test statistique one-way ANOVA.

Les résultats montrent une augmentation significative de 25% de la régénération de la nageoire caudale des larves à 48h après amputation et exposition avec des fils de la composition de tissu objet de la présente invention par rapport aux larves non traitées.

La composition de tissu objet de la présente invention a donc pour effet avantageux de favoriser la régénération de tissus organiques au niveau d'une lésion.

### Etude du recrutement de macrophages

Cette étude a pour objectif d'identifier l'impact de la composition de tissu objet de la présente invention sur le recrutement des macrophages suite à l'amputation de la nageoire caudale chez des larves de *Danio rerio.* Ces larves de *Danio rerio* sont issues d'une lignée transgénique dans laquelle les macrophages expriment la protéine fluorescente verte connue sous le nom GFP pour « *green fluorescent protein* » en terminologie anglo-saxonne.

Les larves sont traitées avec du PTU (1-phenyl 2-thiourea) à partir de 1 jpf.

A 3 jpf, une partie de nageoire caudale des larves est amputée et les larves sont placées dans des plaques 96 puits, une larve par puit. Les larves sont traitées avec des fils du tissu CNI-04 dans la solution contenant le PTU.

Les puits sont ensuite rincés plusieurs fois de sorte à éliminer les fils et permettre une visualisation des résultats sous microscope à fluorescence.

Les résultats sont illustrés en figure 5 sous forme de graphe montrant le recrutement de macrophages au niveau de la région de la blessure (pixel) dans le temps (à 1 heure, 24 heures et 48 heures post-amputation).

Les résultats montrent qu'après exposition à la composition de tissu objet de la présente invention (CNI-04), on observe une augmentation du nombre de macrophages dans la région de la blessure dû à l'amputation dès 1 heure post-amputation par rapport au groupe témoin. Cette augmentation est maintenue à 24 heures et 48 heures post-amputation.

Le traitement avec la composition de tissu objet de la présente invention favorise donc le recrutement de macrophages au niveau de la lésion causée par l'amputation dès les premières heures de régénération.

## Revendications

1. Composition de tissu **caractérisée en ce qu'**elle comprend :
- au moins 50%, de préférence au moins 55%, de préférence au moins 60% de fil en polymère comprenant au moins 1 % de nanoparticules de céramique par rapport à la masse totale du fil, lesdites nanoparticules céramiques étant choisies parmi des nanoparticules de dioxyde de manganèse, d'alumine, d'oxyde de cobalt III, d'oxyde de fer III et de dioxyde de titane ou un mélange d'au moins deux de celles-ci ;
- du fil en polymère dans lequel au moins 100 ppm d'ions argent sont intégrées.

2. Composition de tissu selon la revendication 1, la composition de tissu comportant au moins 50 %, de préférence au moins 55%, préférentiellement au moins 60% de fil de polymère comprenant les nanoparticules de céramique et le fil restant étant du fil de polymère comprenant des ions argent.

3. Composition de tissu selon la revendication 1, comportant entre 30% et 50%, de préférence entre 35% et 45%, préférentiellement entre 35% et 40% de fil de polymère comprenant les ions argent.

4. Composition de tissu selon l'une quelconque des revendications 1 à 3, comportant entre 50% et 70%, de préférence entre 55% et 65%, préférentiellement entre 60% et 65% de fil de polymère comprenant les nanoparticules de céramique.

5. Composition de tissu selon l'une quelconque des revendications 1 à 4, comportant 63% de fil de polymère comprenant les nanoparticules de céramique et 37% de fil de polymère comprenant des ions argent.

6. Composition de tissu selon l'une quelconque des revendications 1 à 5, dans laquelle le fil de polymère comprenant les nanoparticules de céramique constitue la chaîne du tissu et le fil polymère comprenant les ions argent constitue la trame du tissu, ou inversement, le fil de polymère comprenant les nanoparticules de céramique constitue la trame du tissu et le fil polymère comprenant les ions argent constitue la chaîne du tissu.

7. Vêtement ou accessoire comprenant la composition de tissu selon l'une quelconque des revendications 1 à 6, de préférence constitué majoritairement de la composition de tissu selon l'une quelconque des revendications 1 à 6.

8. Equipement de protection individuelle comprenant la composition de tissu selon l'une quelconque des revendications 1 à 6.

9. Pansement ou un bandage comprenant la composition de tissu selon l'une quelconque des revendications 1 à 6.

10. Composition de tissu selon l'une quelconque des revendications 1 à 6, ou vêtement ou accessoire selon la revendication 7, ou équipement de protection individuelle selon la revendication 8, ou pansement ou bandage selon la revendication 9, pour son utilisation en tant que bactéricide et/ou virucide et/ou fongicide.

11. Composition de tissu selon l'une quelconque des revendications 1 à 6, ou vêtement ou accessoire selon la revendication 7, ou équipement de protection individuelle selon la revendication 8, ou pansement ou bandage selon la revendication 9, pour son utilisation dans la prévention d'infection nosocomiale chez le mammifère.

12. Composition de tissu selon l'une quelconque des revendications 1 à 6 ou pansement ou bandage selon la revendication 9, pour son utilisation dans le traitement d'une lésion d'un tissu organique chez le mammifère.

13. Composition de tissu selon l'une quelconque des revendications 1 à 6 ou pansement ou bandage selon la revendication 9, pour son utilisation dans le recrutement de macrophages au niveau d'une lésion de tissus organiques chez le mammifère.

14. Composition de tissu selon l'une quelconque des revendications 1 à 6 ou pansement ou bandage selon la revendication 9, pour son utilisation dans la régénération des chondrocytes.

15. Composition de tissu selon l'une quelconque des revendications 1 à 6 ou pansement ou bandage selon la revendication 9, pour son utilisation dans la régénération nerveuse.

## Patentansprüche

1. Gewebezusammensetzung, die **dadurch gekennzeichnet ist, dass** sie Folgendes umfasst:
- mindestens 50 %, vorzugsweise mindestens 55 %, vorzugsweise mindestens 60 % Polymergarn, das mindestens 1 % keramische Nanopartikel bezogen auf die Gesamtmasse des Garns umfasst, wobei die keramischen Nanopartikel aus Nanopartikeln aus Mangandioxid, Aluminiumoxid, Kobalt-III-Oxid, Eisen-III-Oxid und Titandioxid oder einer Mischung aus mindestens zwei davon ausgewählt werden;
- Polymergarn, in dem mindestens 100 ppm Silberionen integriert sind.

2. Gewebezusammensetzung nach Anspruch 1, wobei die Gewebezusammensetzung mindestens 50 %, vorzugsweise mindestens 55 %, vorzugsweise mindestens 60 % Polymergarn enthält, das die keramischen Nanopartikel umfasst, und wobei das restliche Garn des Polymergarns Silberionen umfasst.

3. Gewebezusammensetzung nach Anspruch 1, die zwischen 30 % und 50 %, vorzugsweise zwischen 35 % und 45 %, vorzugsweise zwischen 35 % und 40 % Polymergarn enthält, das die Silberionen umfasst.

4. Gewebezusammensetzung nach einem der Ansprüche 1 bis 3, das zwischen 50 % und 70 %, vorzugsweise zwischen 55 % und 65 %, vorzugsweise zwischen 60 % und 65 % Polymergarn enthält, das die keramischen Nanopartikel umfasst.

5. Gewebezusammensetzung nach einem der Ansprüche 1 bis 4, die 63 % Polymergarn, das die keramischen Nanopartikel umfasst, und 37 % Polymergarn enthält, das Silberionen umfasst.

6. Gewebezusammensetzung nach einem der Ansprüche 1 bis 5, wobei das Polymergarn, das die keramischen Nanopartikel umfasst, die Kette des Gewebes bildet und das Polymergarn, das die Silberionen umfasst, den Schuss des Gewebes bildet oder umgekehrt, wobei das Polymergarn, das die keramischen Nanopartikel umfasst, den Schuss des Gewebes bildet und das Polymergarn, das die Silberionen umfasst, die Kette des Gewebes bildet.

7. Kleidungsstück oder Zubehörteil, das die Gewebezusammensetzung nach einem der Ansprüche 1 bis 6 umfasst und vorzugsweise größtenteils aus der Gewebezusammensetzung nach einem der Ansprüche 1 bis 6 besteht.

8. Persönliche Schutzausrüstung, die die Gewebezusammensetzung nach einem der Ansprüche 1 bis 6 umfasst.

9. Verband oder Bandage, der bzw. die die Gewebezusammensetzung nach einem der Ansprüche 1 bis 6 umfasst.

10. Gewebezusammensetzung nach einem der Ansprüche 1 bis 6 oder Kleidungsstück oder Zubehörteil nach Anspruch 7 oder persönliche Schutzausrüstung nach Anspruch 8 oder Verband oder Bandage nach Anspruch 9 zur Verwendung als Bakterizid und/oder Viruzid und/oder Fungizid.

11. Gewebezusammensetzung nach einem der Ansprüche 1 bis 6 oder Kleidungsstück oder Zubehörteil nach Anspruch 7 oder persönliche Schutzausrüstung nach Anspruch 8 oder Verband oder Bandage nach Anspruch 9 zur Verwendung bei der Vorbeugung nosokomialer Infektionen bei Säugetieren.

12. Gewebezusammensetzung nach einem der Ansprüche 1 bis 6 oder Verband oder Bandage nach Anspruch 9 zur Verwendung bei der Behandlung einer Läsion eines organischen Gewebes bei Säugetieren.

13. Gewebezusammensetzung nach einem der Ansprüche 1 bis 6 oder Verband oder Bandage nach Anspruch 9 zur Verwendung bei der Rekrutierung von Makrophagen im Bereich einer Läsion von organischem Gewebe bei Säugetieren.

14. Gewebezusammensetzung nach einem der Ansprüche 1 bis 6 oder Verband oder Bandage nach Anspruch 9 zur Verwendung bei der Regeneration von Chondrozyten.

15. Gewebezusammensetzung nach einem der Ansprüche 1 bis 6 oder Verband oder Bandage nach Anspruch 9 zur Verwendung bei der Nervenregeneration.

## Claims

1. A tissue composition **characterized in that** it comprises:
- at least 50%, preferably at least 55%, preferably at least 60% of polymer thread comprising at least 1% of ceramic nanoparticles relative to the total mass of the thread, said ceramic nanoparticles being selected from nanoparticles of manganese dioxide, alumina, cobalt III oxide, iron III oxide and titanium dioxide or a mixture of at least two thereof;
- polymer thread in which at least 100 ppm of silver ions are integrated.

2. The tissue composition according to claim 1, the tissue composition including at least 50%, preferably at least 55%, preferably at least 60% of polymer thread comprising the ceramic nanoparticles and the remaining thread being polymer thread comprising silver ions.

3. The tissue composition according to claim 1, including between 30% and 50%, preferably between 35% and 45%, preferably between 35% and 40% of polymer thread comprising the silver ions.

4. The tissue composition according to any one of claims 1 to 3, including between 50% and 70%, preferably between 55% and 65%, preferably between 60% and 65% of polymer thread comprising the ceramic nanoparticles.

5. The tissue composition according to any one of claims 1 to 4, including 63% of polymer thread comprising the ceramic nanoparticles and 37% of polymer thread comprising silver ions.

6. The tissue composition according to any one of claims 1 to 5, wherein the polymer thread comprising the ceramic nanoparticles constitutes the warp of the tissue and the polymer thread comprising the silver ions constitutes the weft of the tissue, or vice versa, the polymer thread comprising the ceramic nanoparticles constitutes the weft of the tissue and the polymer thread comprising the silver ions constitutes the warp of the tissue.

7. Garment or accessory comprising the tissue composition according to any one of claims 1 to 6, preferably consisting mainly of the tissue composition according to any one of claims 1 to 6.

8. Personal protective equipment comprising the tissue composition according to any one of claims 1 to 6.

9. A dressing or a bandage comprising the tissue composition according to any one of claims 1 to 6.

10. The tissue composition according to any one of claims 1 to 6, or garment or accessory according to claim 7, or personal protective equipment according to claim 8, or dressing or bandage according to claim 9, for its use as a bactericide and/or a virucide and/or a fungicide.

11. The tissue composition according to any one of claims 1 to 6, or garment or accessory according to claim 7, or personal protective equipment according to claim 8, or dressing or bandage according to claim 9, for its use in the prevention of nosocomial infection in the mammal.

12. The tissue composition according to any one of claims 1 to 6 or dressing or bandage according to claim 9, for its use in the treatment of an injury to an organic tissue in the mammal.

13. The tissue composition according to any one of claims 1 to 6 or dressing or bandage according to claim 9, for its use in the recruitment of macrophages at an organic tissue injury in the mammal.

14. The tissue composition according to any one of claims 1 to 6 or dressing or bandage according to claim 9, for its use in the regeneration of the chondrocytes.

15. The tissue composition according to any one of claims 1 to 6 or dressing or bandage according to claim 9, for its use in nerve regeneration.
